# EUROPEAN PATENT APPLICATION

(11) **EP 3 584 673 A1**
(43) Date of publication of application: **25.12.2019**
(21) Application number: 18753528.1
(22) Date of filing: 02.02.2018
(51) Int. Cl.: G06F 1/26, A61B 5/0402, G06F 1/32, G06F 3/01

(54) **WEARING DETERMINATION DEVICE AND ELECTRONIC DEVICE**

(30) Priority: 17.02.2017 JP 2017028540
(71) Applicant: Alps Alpine Co., Ltd., Tokyo 145-8501 (JP)
(72) Inventor: NAKAMURA Toshiki, Tokyo 145-8501 (JP); YAMADA Yukimitsu, Tokyo 145-8501 (JP); TAKAI Daisuke, Tokyo 145-8501 (JP); TOKITA Seiji, Tokyo 145-8501 (JP); SASAKI Takashi, Tokyo 145-8501 (JP)
(74) Representative: Maiwald Patent- und Rechtsanwaltsgesellschaft mbH
(86) International application number: PCT/JP2018/003645
(87) International publication number: WO 2018/150914

(57) **Abstract**

[Object] A wearing determination apparatus that accurately determines whether an electronic device is worn to achieve power saving is provided.

[Solution] A wakeup process of a living body tactile sensor 13 and a wearing determination unit 21 is performed when an accelerometer 11 detects an acceleration. Accordingly, the living body tactile sensor 13 and the wearing determination unit 21 are in a sleep mode while the accelerometer 11 detects no acceleration. When the wearing determination unit 21 determines a non-wearing state, the wearing determination unit 21 performs a sleep process to switch itself from a wakeup mode to the sleep mode.

## Description

### Technical Field

The present invention relates to a wearing determination apparatus and an electronic device that uses the wearing determination apparatus.

### Background Art

PTL 1 discloses a technique for reducing a power consumption in a battery-powered electronic device when not in use.

The electronic device detects whether a hand of a user approaches in a sleep mode and becomes active when it is determined that the hand approaches.

PTL 1 also discloses that a wakeup process is performed when an acceleration is detected in the sleep mode.

### Citation List

### Patent Literature

PTL 1: Japanese Unexamined Patent Application Publication No. 2004-240971

### Summary of Invention

### Technical Problem

As disclosed in PTL 1 described above, however, in the case where the wakeup process from sleep is performed only due to approach of the user or wearing determination, it is necessary to continue the wearing determination or approach with a relatively high power consumption during a sleep period, and the power consumption is high. In some cases where a conductor other than a living body approaches or comes into contact, the wakeup process is unnecessarily performed.

In the case where the wakeup process is started only under the condition that the electronic device is accelerated, the wakeup process is performed even when the electronic device is not worn on the living body and the electronic device vibrates.

The present invention has been accomplished in view of such circumstances, and an object of the present invention is to provide a wearing determination apparatus that can accurately determine whether an electronic device is worn, and the electronic device that achieves power saving by using the wearing determination apparatus.

### Solution to Problem

In order to solve the above problem of the related art and achieve the above object, a wearing determination apparatus according to the present invention is a wearing determination apparatus configured to be installed in an electronic device configured to be worn on a living body. The wearing determination apparatus includes an accelerometer, a tactile sensor including a living body contact portion configured to come into contact with the living body, the tactile sensor generating a signal in response to contact between the living body contact portion and the living body, and a wearing determination unit configured to determine whether the living body contact portion is in contact with the living body on the basis of a signal from the accelerometer and the tactile sensor. The accelerometer outputs a first interrupt signal when the accelerometer detects an acceleration. The tactile sensor and the wearing determination unit perform a wakeup process to switch themselves from a sleep mode to a wakeup mode in response to the first interrupt signal outputted from the accelerometer.

With this structure, the wakeup process of the tactile sensor and the wearing determination unit is performed when the accelerometer detects the acceleration. Accordingly, the tactile sensor and the wearing determination unit can be in the sleep mode while the accelerometer detects no acceleration, and power saving can be achieved.

In addition, with this structure, a wearing determination can be made by the wearing determination unit in addition to detection of the acceleration by the accelerometer. Accordingly, the wakeup process of another circuit block can be performed under the condition that wearing on the living body is determined. The wakeup process of the circuit block can be prevented from being performed in a non-wearing state. This also achieves power saving.

In addition, with this structure, the wakeup process of the circuit block can be performed under the condition that the accelerometer detects the acceleration in addition to the wearing determination, and the wakeup process can be prevented from being unnecessarily performed when a conductor other than the living body comes into contact with the living body contact portion. This also achieves power saving.

The wearing determination unit preferably determines whether the living body contact portion is in contact with the living body on the basis of a signal from the tactile sensor after the wakeup process and performs a wakeup process to cause a predetermined circuit block of the electronic device to switch from the sleep mode to the wakeup mode in a case of a positive determination.

With this structure, the wearing determination can be made by the wearing determination unit in addition to detection of the acceleration by the accelerometer. Accordingly, the wakeup process of another circuit block can be performed under the condition that wearing on the living body is determined. The wakeup process of the circuit block can be prevented from being performed in a non-wearing state. This also achieves power saving.

The wearing determination unit preferably performs a sleep process to switch itself from the wakeup mode to the sleep mode when the wearing determination unit determines that the living body contact portion is not in contact with the living body.

With this structure, the wearing determination unit can be in the sleep mode in the non-wearing state and power saving is achieved.

The accelerometer preferably outputs a second interrupt signal when the accelerometer detects no acceleration for a certain period of time.

The tactile sensor and the wearing determination unit preferably perform a sleep process to switch themselves from the wakeup mode to the sleep mode in response to the second interrupt signal.

With this structure, the tactile sensor and the wearing determination unit are in the sleep mode when no acceleration is detected for a certain period of time. This achieves power saving.

A power consumption of the accelerometer is preferably lower than a power consumption of the tactile sensor and the wearing determination unit in the wakeup mode.

With this structure, the wearing determination unit and the tactile sensor that have a relatively high power consumption can be in the sleep mode.

A power consumption of the wearing determination unit in the wakeup mode is preferably lower than a power consumption of the predetermined circuit block in the wakeup mode.

With this structure, the circuit block that has a relatively high power consumption can be in the sleep mode.

An electronic device according to the present invention is an electronic device configured to be worn on a living body. The electronic device includes the wearing determination apparatus. The wearing determination apparatus includes an accelerometer, a tactile sensor including a living body contact portion configured to come into contact with the living body, the tactile sensor generating a signal in response to contact between the living body contact portion and the living body, and a wearing determination unit configured to determine whether the living body contact portion is in contact with the living body on the basis of a signal from the accelerometer and the tactile sensor. The accelerometer outputs a first interrupt signal when the accelerometer detects an acceleration. The tactile sensor and the wearing determination unit perform a wakeup process to switch themselves from a sleep mode to a wakeup mode in response to the first interrupt signal outputted from the accelerometer.

With this structure, the wakeup process of the tactile sensor and the wearing determination unit is performed when the accelerometer detects the acceleration. Accordingly, the tactile sensor and the wearing determination unit can be in the sleep mode while the accelerometer detects no acceleration, and power saving can be achieved.

In addition, with this structure, the wearing determination can be made by the wearing determination unit in addition to detection of the acceleration by the accelerometer. Accordingly, the wakeup process of another circuit block that is included in the electronic device can be performed under the condition that wearing on the living body is determined. The wakeup process of the circuit block can be prevented from being performed in a non-wearing state. This also achieves power saving.

In addition, with this structure, the wakeup process of the circuit block can be performed under the condition that the accelerometer detects the acceleration in addition to the wearing determination, and the wakeup process can be prevented from being unnecessarily performed when a conductor other than the living body comes into contact with the living body contact portion. This also achieves power saving.

### Advantageous Effects of Invention

The present invention can provide a wearing determination apparatus that can accurately determine whether an electronic device is worn, and the electronic device that achieves power saving by using the wearing determination apparatus.

### Brief Description of Drawings

[Fig. 1] Fig. 1 illustrates the structure of a living body information measurement apparatus according to an embodiment of the present invention.
[Fig. 2] Fig. 2 is a functional block diagram of a control system of the living body information measurement apparatus illustrated in Fig. 1.
[Fig. 3] Fig. 3 is a flowchart for describing a wakeup process of the living body information measurement apparatus illustrated in Fig. 1.
[Fig. 4] Fig. 4 is a flowchart for describing a primary wakeup process at a step ST5 illustrated in Fig. 3.
[Fig. 5] Fig. 5 is a flowchart for describing a sleep process of the living body information measurement apparatus illustrated in Fig. 1.

### Description of Embodiments

### (Overall Structure)

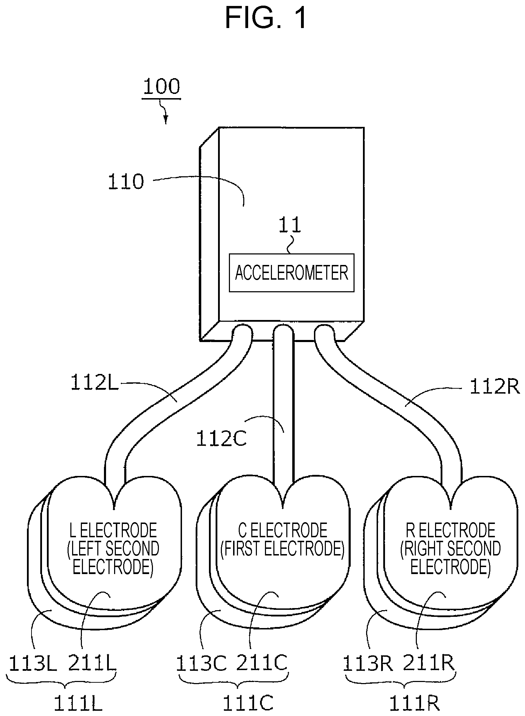

A living body information measurement apparatus according to an embodiment of the present invention will hereinafter be described. Fig. 1 illustrates the structure of a living body information measurement apparatus 100 according to an embodiment of the present invention. The living body information measurement apparatus 100 includes a control device 110, a C pad 111C, a L pad 111L, a R pad 111R, a C wiring line 112C, a L wiring line 112L, and a R wiring line 112R.

The C pad 111C includes a flat-plate-shaped C insulator 113C and a flat-plate-shaped first electrode 211C (also referred to as a common electrode, and referred to below as a C electrode 211C in some cases) that is stuck to a surface of the C insulator 113C. The L pad 111L includes a flat-plate-shaped L insulator 113L and a left second electrode 211L (referred to below as a L electrode 211L in some cases) that is stuck to a surface of the L insulator 113L. The R pad 111R includes a flat-plate-shaped R insulator 113R and a right second electrode 211R (referred to below as a R electrode 211R in some cases) that is stuck to a surface of the R insulator 113R. In the following description, the C electrode 211C, the L electrode 211L, and the R electrode 211R are not distinguished from each other and are referred to electrodes 211 in some cases. Each of the electrodes 211 is composed of a conductive material such as a metal and exposed to the outside so as to be capable of coming into contact with a living body.

The control device 110 accommodates components that are included in an electrical system described later. The C wiring line 112C connects the control device 110 and the C electrode 211C to each other. The L wiring line 112L connects the control device 110 and the L electrode 211L to each other. The R wiring line 112R electrically connects the control device 110 and the R electrode 211R to each other.

The living body information measurement apparatus 100 carries out living body information measurement to measure living body information and wearing state detection to detect a wearing state of one or more electrodes 211 against the living body on the basis of an electrical signal from the living body. An example of the living body is a human body. An example of the living body information is an electrocardiogram. The living body information measurement apparatus 100 operates by using the power of a built-in battery not illustrated.

The C electrode 211C, the L electrode 211L, and the R electrode 211R are disposed on a skin of the human body near the heart. The C electrode 211C is disposed nearest to the heart between the L electrode 211L and the R electrode 211R. The L electrode 211L and the R electrode 211R are disposed symmetrically about the C electrode 211C when being properly worn on the living body. A voltage waveform between the C electrode 211C and the L electrode 211L is detected. In addition, a voltage waveform between the C electrode 211C and the R electrode 211R is detected. The difference between the two detected voltage waveforms represents electrocardiogram information of the human body.

### (Structure of Control System)

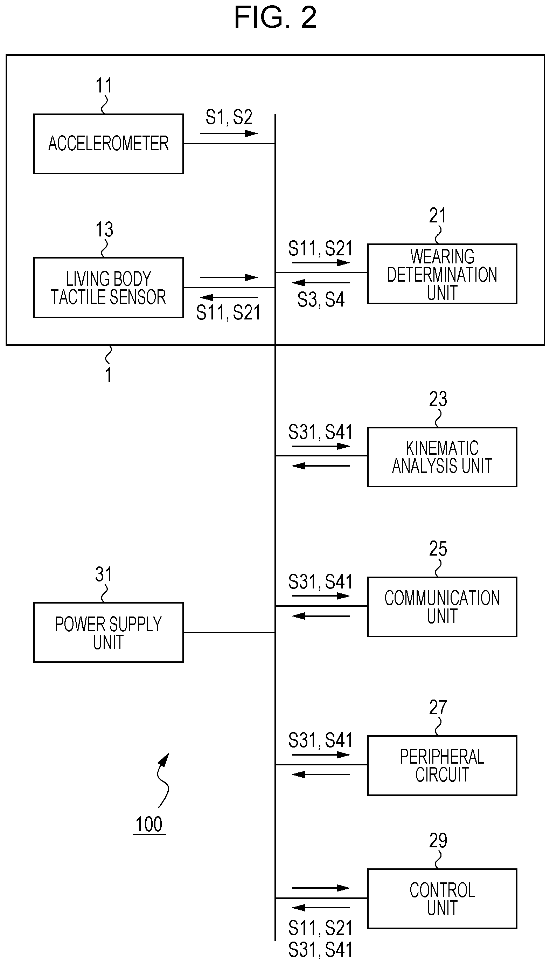

Fig. 2 is a functional block diagram of a control system of the living body information measurement apparatus 100 illustrated in Fig. 1.

As illustrated in Fig. 1, the living body information measurement apparatus 100 includes, for example, an accelerometer 11, a living body tactile sensor 13, a wearing determination unit 21, a kinematic analysis unit 23, a communication unit 25, a peripheral circuit 27, a control unit 29, and a power supply unit 31.

The accelerometer 11, the living body tactile sensor 13, and the wearing determination unit 21 are included in a wearing determination apparatus 1 according to the embodiment of the present invention.

The accelerometer 11 is installed, for example, in a control device 100 illustrated in Fig. 1. The accelerometer 11 generates an acceleration signal. The accelerometer 11 outputs a first interrupt signal S1 to the control unit 29 when an acceleration is detected in a state where no acceleration is created for a certain period of time.

The control unit 29 outputs a first interrupt signal S11 depending on the first interrupt signal S1 to the living body tactile sensor 13 and the wearing determination unit 21.

The accelerometer 11 outputs a second interrupt signal S2 to the control unit 29 when no acceleration is detected for a certain period of time after the acceleration is detected.

The control unit 29 outputs a second interrupt signal S21 depending on the second interrupt signal S2 to the living body tactile sensor 13 and the wearing determination unit 21.

The living body tactile sensor 13 includes the C pad 111C, the L pad 111L, and the R pad 111R, which are living body contact portions that come into contact with the living body, illustrated in Fig. 1. The living body tactile sensor 13 also includes a signal applicator (not illustrated) and a signal amplifier (not illustrated).

The living body tactile sensor 13 can select a wakeup mode in which electrical characteristics of each living body contact portion are detected and a sleep mode in which the power consumption is lower than that in the wakeup mode.

When the first interrupt signal S11 is inputted from the control unit 29 in the sleep mode, the living body tactile sensor 13 performs its own wakeup process to switch to the wakeup mode.

When the second interrupt signal S21 is inputted from the control unit 29 in the wakeup mode, the living body tactile sensor 13 performs its own sleep process to switch to the sleep mode.

In the wakeup mode, the wearing determination unit 21 determines whether the living body information measurement apparatus 100 is worn on the living body on the basis of the signal from the living body tactile sensor 13.

When the wearing determination unit 21 determines that the living body information measurement apparatus 100 is worn on the living body and the kinematic analysis unit 23, the communication unit 25, and the peripheral circuit 27 are in the sleep mode, the wearing determination unit 21 transmits a third interrupt signal S3 to the control unit 29. The control unit 29 outputs a third interrupt signal S31 depending on the third interrupt signal S3 to the kinematic analysis unit 23, the communication unit 25, and the peripheral circuit 27, which perform their own wakeup process.

The wearing determination unit 21 may make a wearing determination by using the signal from the accelerometer 11 in addition to the signal from the living body tactile sensor 13. The wearing determination with the signal from the living body tactile sensor 13 can be made more accurately than that with the acceleration signal from the accelerometer 11.

When the wearing determination unit 21 determines that the living body information measurement apparatus 100 is not worn on the living body, the wearing determination unit 21 outputs a fourth interrupt signal S4 to the control unit 29. The control unit 29 outputs a fourth interrupt signal S41 depending on the fourth interrupt signal S4 to the kinematic analysis unit 23, the communication unit 25, and the peripheral circuit 27. Consequently, the kinematic analysis unit 23, the communication unit 25, and the peripheral circuit 27 perform their own sleep process.

The power consumption of the living body tactile sensor 13 and the wearing determination unit 21 in the wakeup mode is higher than the power consumption of the accelerometer 11.

The kinematic analysis unit 23 performs a kinematic analysis process of a wearer of the living body information measurement apparatus 100 on the basis of the signals from the accelerometer 11 and the living body tactile sensor 13.

When the third interrupt signal S31 is inputted from the control unit 29, the kinematic analysis unit 23 performs its own wakeup process. When the fourth interrupt signal S41 is inputted from the control unit 29, the kinematic analysis unit 23 performs its own sleep process.

The communication unit 25 has a communication function of transmitting, for example, the result of the wearing determination of the wearing determination unit 21 and the result of kinematic analysis of the kinematic analysis unit 23 to a predetermined communication device.

When the third interrupt signal S31 is inputted from the control unit 29, the communication unit 25 performs its own wakeup process. When the fourth interrupt signal S41 is inputted from the control unit 29, the communication unit 25 performs its own sleep process.

The peripheral circuit 27 has a function of performing processes such as a process of monitoring supply voltage of the power supply unit and a process of making a LED indicator capable of being emitted.

When the third interrupt signal S31 is inputted from the control unit 29, the peripheral circuit 27 performs its own wakeup process. When the fourth interrupt signal S41 is inputted from the control unit 29, the peripheral circuit 27 performs its own sleep process.

The power consumption of the kinematic analysis unit 23, the communication unit 25, and the peripheral circuit 27 in the wakeup mode is higher than, but may be equal to or lower than, the power consumption of the living body tactile sensor 13 and the wearing determination unit 21 in the wakeup mode.

An example of operation related to the wakeup process and the sleep process of the living body information measurement apparatus 100 will now be described.

### [Wakeup Process]

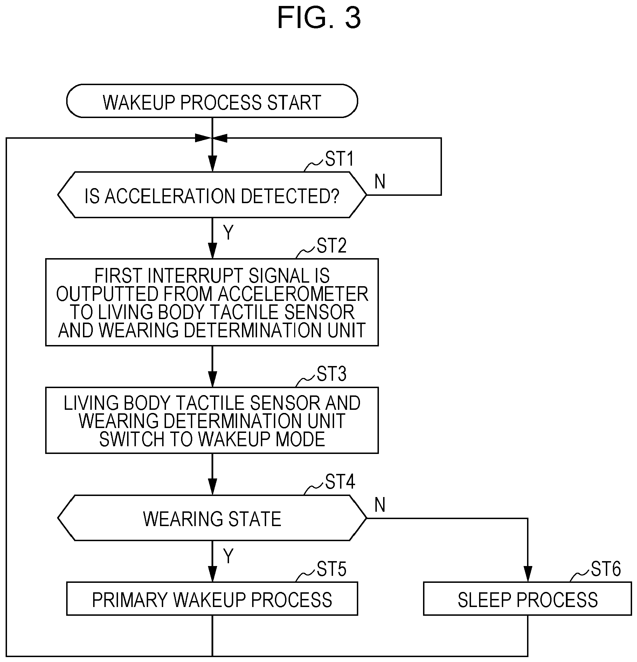

Fig. 3 is a flowchart for describing the wakeup process of the living body information measurement apparatus 100 illustrated in Fig. 1.

### Step ST1:

The accelerometer 11 determines whether an acceleration is created in a state where the living body information measurement apparatus 100 is not accelerated for a certain period of time. In the case of a positive determination, the flow proceeds to a step ST12. In the case of a negative determination, the determination is repeated.

### Step ST2:

The accelerometer 11 outputs the first interrupt signal S1 to the control unit 29. The control unit 29 outputs the first interrupt signal S11 depending on the first interrupt signal S1 to the living body tactile sensor 13 and the wearing determination unit 21.

### Step ST3:

When the first interrupt signal S11 is inputted from the control unit 29 in the sleep mode, the living body tactile sensor 13 performs its own wakeup process to switch to the wakeup mode.

When the first interrupt signal S11 is inputted from the control unit 29 in the sleep mode, the wearing determination unit 21 performs its own wakeup process to switch to the wakeup mode.

Consequently, the wearing determination unit 21 performs a wearing determination process to determine whether the living body information measurement apparatus 100 is worn on the living body on the basis of the signal of the living body tactile sensor 13.

### Step ST4:

When the result of the wearing determination process of the wearing determination unit 21 is the positive determination, that is, a wearing state, the flow proceeds to a step ST5. In the case of the negative determination, the flow proceeds to a step ST6.

### Step ST5:

The wearing determination unit 21 outputs the third interrupt signal S3 to the control unit 29.

The control unit 29 outputs the third interrupt signal S31 depending on the third interrupt signal S3 to the kinematic analysis unit 23, the communication unit 25, and the peripheral circuit 27.

The kinematic analysis unit 23, the communication unit 25, and the peripheral circuit 27 perform the wakeup process in response to the third interrupt signal S31 to switch to the wakeup mode.

### Step ST6:

The wearing determination unit 21 performs its own sleep process to switch to the sleep mode.

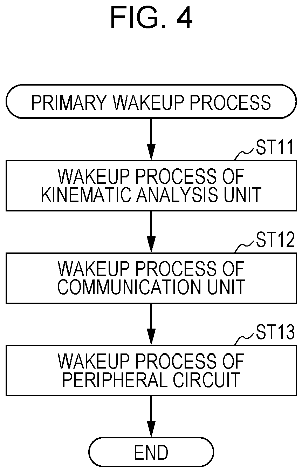

Fig. 4 is a flowchart for describing the primary wakeup process at the step ST5 illustrated in Fig. 3.

As illustrated in Fig. 4, the kinematic analysis unit 23 performs the wakeup process on the basis of the third interrupt signal S31 from the control unit 29 to switch to the wakeup mode (step ST11). Consequently, the kinematic analysis unit 23 performs the kinematic analysis process of the wearer of the living body information measurement apparatus 100 on the basis of the signals from the accelerometer 11 and the living body tactile sensor 13.

Subsequently, the communication unit 25 performs the wakeup process on the basis of the third interrupt signal S31 from the control unit 29 to switch to the wakeup mode (step ST12). Consequently, the communication unit 25 performs a communication process of transmitting, for example, the result of the wearing determination of the wearing determination unit 21 and the result of the kinematic analysis of the kinematic analysis unit 23 to a predetermined communication device.

Subsequently, the peripheral circuit 27 performs the wakeup process on the basis of the third interrupt signal S31 from the control unit 29 to switch to the wakeup mode (step ST13).

### Step ST6:

The wearing determination unit 21 performs its own sleep process to switch to the sleep mode.

### [Sleep Process]

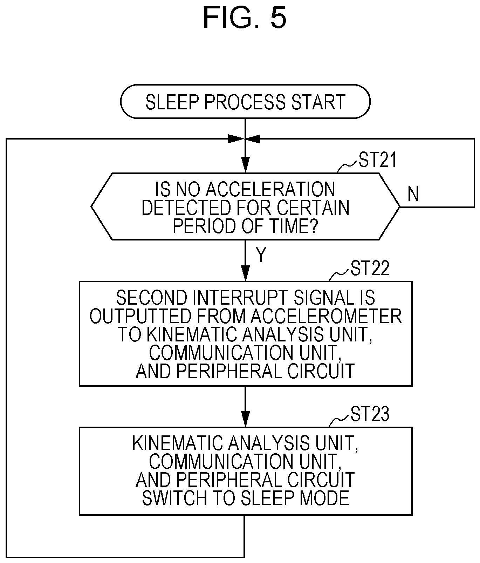

Fig. 5 is a flowchart for describing the sleep process of the living body information measurement apparatus 100 illustrated in Fig. 1.

### Step ST21:

When the accelerometer 11 determines that no acceleration is detected for a certain period of time after the acceleration is detected, the flow proceeds to a step ST22. When this is not the case, the determination is repeated.

### Step ST22:

The accelerometer 11 outputs the second interrupt signal S2 to the control unit 29.

The control unit 29 outputs the second interrupt signal S21 depending on the second interrupt signal S2 to the living body tactile sensor 13, the wearing determination unit 21, the kinematic analysis unit 23, the communication unit 25, and the peripheral circuit 27.

### Step ST23:

The living body tactile sensor 13, the wearing determination unit 21, the kinematic analysis unit 23, the communication unit 25, and the peripheral circuit 27 perform the sleep process in response to the second interrupt signal S21 to switch to the sleep mode.

As described above, the living body information measurement apparatus 100 performs the wakeup process of the living body tactile sensor 13 and the wearing determination unit 21 when the accelerometer 11 detects the acceleration. Accordingly, the living body tactile sensor 13 and the wearing determination unit 21 can be in the sleep mode while the accelerometer 11 detects no acceleration, and power saving can be achieved.

The living body information measurement apparatus 100 can make the wearing determination by the living body tactile sensor 13 and the wearing determination unit 21 in addition to detection of the acceleration by the accelerometer 11. Accordingly, the wakeup process of the kinematic analysis unit 23, the communication unit 25, and the peripheral circuit 27, which are the other circuit blocks, can be performed under the condition that wearing on the living body is determined. The wakeup process of the circuit blocks can be prevented from being performed in a non-wearing state. This also achieves power saving.

The living body information measurement apparatus 100 can perform the wakeup process of the kinematic analysis unit 23, the communication unit 25, and the peripheral circuit 27 under the condition that the accelerometer 11 detects the acceleration in addition to the wearing determination by the kinematic analysis unit 23 and can prevent the wakeup process from being unnecessarily performed when a conductor comes into contact with the R electrode 211R, the L electrode 211L, or the C electrode 211C, which is the living body contact portion. This also achieves power saving.

The living body information measurement apparatus 100 performs the sleep process to switch itself from the wakeup mode to the sleep mode when the wearing determination unit 21 determines the non-wearing state. This enables the wearing determination unit 21 to be in the sleep mode in the non-wearing state and achieves power saving.

The living body information measurement apparatus 100 outputs the second interrupt signal when the accelerometer 11 detects no acceleration for a certain period of time. In response to this, the living body tactile sensor 13 and the wearing determination unit 21 perform the sleep process to switch themselves from the wakeup mode to the sleep mode. This also achieves power saving.

The present invention is not limited to the above embodiment.

That is, a person skilled in the art may make various modifications, combinations, sub-combinations, and alternations regarding the components according to the above embodiment within the technical range or the equivalent range of the present invention.

For example, according to the above embodiment, a glasses electronic device is described as an example of an electronic device according to the present invention. However, another electronic device that is worn on the human body such as a list band or a watch may be acceptable.

According to the above embodiment, the human body is described as an example of the living body. However, the case of wearing on the living body of, for example, an animal such as a pet other than the human body is also acceptable.

In an example described according to the above embodiment, the living body tactile sensor 13 and the wearing determination unit 21 are different modules but may be a single module.

According to the above embodiment, the kinematic analysis unit 23, the communication unit 25, and the peripheral circuit 27 are described as examples of circuit blocks according to the present invention. However, other circuits may be acceptable.

### Industrial Applicability

The present invention can be applied to various living body information measurement apparatuses that are worn on the living body, for example, living body information measurement apparatuses that are used for electrocardiogram measurement, eye potential measurement, and muscle potential measurement.

### Reference Signs List

- 1: wearing determination apparatus
- 11: accelerometer
- 13: living body tactile sensor
- 21: wearing determination unit
- 23: kinematic analysis unit
- 25: communication unit
- 27: peripheral circuit
- 29: control unit
- 31: power supply unit
- 100: living body information measurement apparatus
- 111C: C pad
- 111L: L pad
- 111R: R pad
- 113C: C insulator
- 113L: L insulator
- 113R: R insulator
- 211C: C electrode
- 211L: L electrode
- 211R: R electrode

## Claims

1. A wearing determination apparatus configured to be installed in an electronic device configured to be worn on a living body, the wearing determination apparatus comprising:
an accelerometer;
a tactile sensor including a living body contact portion configured to come into contact with the living body, the tactile sensor generating a signal in response to contact between the living body contact portion and the living body; and
a wearing determination unit configured to determine whether the living body contact portion is in contact with the living body on the basis of a signal from the accelerometer and the tactile sensor,
wherein the accelerometer outputs a first interrupt signal when the accelerometer detects an acceleration, and
wherein the tactile sensor and the wearing determination unit perform a wakeup process to switch themselves from a sleep mode to a wakeup mode in response to the first interrupt signal outputted from the accelerometer.

2. The wearing determination apparatus according to Claim 1,
wherein the wearing determination unit determines whether the living body contact portion is in contact with the living body on the basis of the signal from the tactile sensor after the wakeup process and performs a wakeup process to cause a predetermined circuit block of the electronic device to switch from the sleep mode to the wakeup mode in a case of a positive determination.

3. The wearing determination apparatus according to Claim 1,
wherein the wearing determination unit performs a sleep process to switch itself from the wakeup mode to the sleep mode when the wearing determination unit determines that the living body contact portion is not in contact with the living body.

4. The wearing determination apparatus according to Claim 1,
wherein the accelerometer outputs a second interrupt signal when the accelerometer detects no acceleration for a certain period of time, and
wherein the tactile sensor and the wearing determination unit perform a sleep process to switch themselves from the wakeup mode to the sleep mode in response to the second interrupt signal.

5. The wearing determination apparatus according to Claim 1,
wherein a power consumption of the accelerometer is lower than a power consumption of the tactile sensor and the wearing determination unit in the wakeup mode.

6. The wearing determination apparatus according to Claim 2,
wherein a power consumption of the wearing determination unit in the wakeup mode is lower than a power consumption of the predetermined circuit block in the wakeup mode.

7. An electronic device configured to be worn on a living body, the electronic device comprising:
a wearing determination apparatus that includes an accelerometer,
a tactile sensor including a living body contact portion configured to come into contact with the living body, the tactile sensor generating a signal in response to contact between the living body contact portion and the living body, and
a wearing determination unit configured to determine whether the living body contact portion is in contact with the living body on the basis of a signal from the accelerometer and the tactile sensor,
wherein the accelerometer outputs a first interrupt signal when the accelerometer detects an acceleration, and
wherein the tactile sensor and the wearing determination unit perform a wakeup process to switch themselves from a sleep mode to a wakeup mode in response to the first interrupt signal outputted from the accelerometer.
